# EUROPEAN PATENT SPECIFICATION

(11) **EP 3 320 936 B1**
(45) Date of publication and mention of the grant of the patent: **03.11.2021**
(21) Application number: 17200531.6
(22) Date of filing: 08.11.2017
(51) Int. Cl.: A61M 15/00

(54) **A NEW GUIDE GEAR FOR INHALER DEVICES**
NEUE FÜHRUNGSVORRICHTUNG FÜR INHALATIONSGERÄTE
NOUVELLE ROUE DE GUIDAGE POUR DES DISPOSITIFS D'INHALATION

(30) Priority: 09.11.2016 TR 201616027
(43) Date of publication of application: 16.05.2018
(73) Proprietor: Arven Ilac Sanayi Ve Ticaret A.S., Istanbul 34460 (TR)
(72) Inventor: TOKSÖZ, Ahmet, 34460 Istanbul (TR); TOKSÖZ, Zafer, 34460 Istanbul (TR)
(74) Representative: Mutlu, Aydin

(56) References cited:
- EP-A1- 2 239 001
- GB-A- 2 457 615
- US-A1- 2005 103 337
- US-A1- 2010 258 118
- US-A1- 2010 307 492

## Description

### Technical Field

The present invention relates to improvements in gear or wheel members of the inhaler device that provides the administration of dry powder inhalation medicament.

### Prior Art

Despite the advances in diagnosis and treatment in recent years, diseases such as asthma, bronchitis and COPD (Chronic Obstructive Pulmonary Disease) decrease the quality of life of humans. The administration of medicaments by means of inhalers is recommended for optimizing the treatment of these types of diseases. The treatment by means of inhalers is the most preferred treatment type and is expected to be the first option in the future. The most important advantage of using medicaments by means of inhalation is to provide a more efficient treatment by using less medicaments, to administer medicaments in a higher concentration the respiratory tract and in particular to decrease the systemic side effects of medicaments. Despite the presence of very efficient treatments for respiratory tract diseases, the most significant reasons of failing to properly control patients are stated as noncompliance with the treatments recommended by the physicians and incoordination due to the improper use of inhalers.

In recent years, various inhalation devices have been developed for administering inhalation medicaments. Said devices are basically categorized into two types: metered-dose inhalers and dry powder inhalers. These types of devices structurally comprise basic components such as mechanism, counter, body, lid, lock, etc. In addition, powder inhalation medicaments are preserved in carriers such as blister, capsule, etc. Blisters are composed of two basic components, namely the main layer with cavities for carrying the medicament and the strippable lid layer.

The types of inhaler devices that comprise a plurality of blisters contain therein various movement and force transmission components. Said components are generally articulated to each other and positioned in a protective body. The body is generally composed of two complementary parts. One of said parts is positioned under said components so as to form a base for the same and the other is positioned thereabove so as to form a lid. The movement and force transmission components positioned between the parts of the body are designed so as to optimize the use of the medicament-carrying blisters. Said movement and force transmission components comprise gears and wheels of various types and in various dimensions. By means of the harmonious operation of said gears and wheels, the use of medicaments becomes possible.

Particularly in blister inhaler devices, there is provided a guide gear that guides the cavities filled with medicaments towards the mouthpiece and positions the same for optimum use when said blisters are in a position ready for use, in other words when the cavities thereof are open. The blister cavities are fitted into the gaps between the teeth on the body of the guide gear. With the movement of the mechanism, the blisters are opened and positioned when between two teeth such that the medicament is aligned with the outlet hole. Particularly in patent documents no. EP2239002 B1 and EP2666498 A1, samples of said guide gears can be found. However, said guide gears pose various problems. As can be seen in said two state of the art patent documents, due to the operation principle of the inhaler devices, the blister having cavities filled with medicaments moves unidirectionally and the used-up, empty blister cavities are stored at the inner section of the device. The unidirectional movement of mechanism in said devices is realized by means of the recessed surface inclined in one direction at the inner section of said guide gear as well as a second gear that is disposed into said guide gear and that has angled tabs. Said guide gear and the gear with angled tab engage with each other. The recessed surface in the guide gear and the angled tab of the second gear disposed into said guide gear allow unidirectional movement. However, problems arise from the unidirectional rotation at the inner section of said guide gear. Due to the centrifugal force generated during said rotational movement, the upper section of the guide gear wobbles out of the axis of movement. This wobbling causes the blister to dislodge from the movement channel, thus preventing the device from operating with the desired efficiency. Moreover, said gear can be provided in the form of a ratchet having inclined/angled teeth enabling the unidirectional movement and an arm acting as a latch. In this case, the blister would slide left and right on the inclined teeth and the desired efficiency could not be obtained. Such an error would cause the blister to dislodge from the channels wherein the same moves such that the medicament would spill, that the medicament could not be used in the ideal dose and that the mechanism could be jammed.

US2010/0307492 A discloses a dry powder inhaler device comprising a guide wheel comprising recesses for accepting blisters of a blister strip, and a profile co-operating with a pawl. GB2457615 A discloses another dry powder inhaler device comprising a magazine member with dose compartments and with teeth co-operating with a pawl.

### Aim and Brief Description of the Invention

The present invention is defined in the appended independent claim 1 and relates to a new guide gear used in dry powder inhaler devices, that eliminates all the above-mentioned problems and that provides advantages to the relevant technical field. Preferred embodiments are matter of the dependent claims.

With respect to the state of the art, the principal aim of the present invention is to provide a guide gear that can be operated with precision in a dry powder inhaler device.

Another aim of the present invention is to provide a guide gear that does not wobble out of the central axis during the rotational movement in a dry powder inhaler device and that prevents the medicament-carrying blister from dislodging from the channel wherein the same moves.

Yet another aim of the present invention is to provide a gear assembly that allows the use of minimum volume.

Yet another aim of the present invention is to provide a dry powder inhaler device with a blister advancement mechanism, that smoothly operates by means of the guide gear preventing jamming and breaking.

In order to attain all the aims that are described above and that may arise from the below detailed description,
An inhaler device is developed, that comprises a ratchet and a corresponding pawl engaging the ratchet to rotate in the same direction wherein an enlarged outer periphery of the teeth profile of the ratchet is enabled such that holding blister in a secure manner while rotation of the ratchet.

According to the present invention, a circular guide gear is positioned opposite to a locking arm/pawl in the movement mechanism, wherein blister cavities filled with powder medicament are fitted in between the teeth provided on the body of said guide gear.

The invention is realized by shaping the teeth of said guide gear in two or more different forms.

In another preferred embodiment of the present invention, said teeth having two different forms are provided with one of the flat, outward/inward oval, castellated, inclined/radius or conic forms.

In yet another preferred embodiment of the present invention, one section of said teeth having two different forms has an inclined/radius surface and the other section thereof has a flat surface.

In yet another preferred embodiment of the present invention, the longitudinal ratio of the inclined/radius surface section of said teeth to the flat surface section is in the range of 0.1 - 9.

In yet another preferred embodiment of the present invention, the longitudinal ratio of the inclined/radius surface section of said teeth to the flat surface section is in the range of 1 - 5.

In yet another preferred embodiment of the present invention, the ratio of the inclined/radius surface section of said teeth to the flat surface section is in the range of 1.3 - 3.

In yet another preferred embodiment of the present invention, the ratio of the inclined/radius surface section of said teeth to the flat surface section is in the range of 1.5 - 2.

In yet another preferred embodiment of the present invention, each of the teeth of said guide gear has the same structure and said teeth are shaped in two or more different forms.

In yet another preferred embodiment of the present invention, the cross-sectional area of the teeth of said guide gear is selected from rectangular, square, oval, radius, wave forms or from the combination of these forms.

### Brief Description of Figures

Figure 1 shows the view of a representative embodiment of the state of the art.
Figure 2 shows the perspective view of a representative embodiment of the inhaler device of the present invention.
Figure 3 shows the representative perspective view of the inhaler device of the present invention in the disassembled state.
Figure 4 shows the representative perspective view of the inhaler device of the present invention in the disassembled state.
Figure 5 shows the detailed perspective view of the guide gear disposed in the inhaler device of the present invention.
Figure 6a shows the sideways view of a representative embodiment of the guide gear disposed in the inhaler device of the present invention.
Figure 6b shows the top view of a representative embodiment of the guide gear disposed in the inhaler device of the present invention.
Figure 7 shows the perspective view of a representative embodiment of the guide gear disposed in the inhaler device of the present invention.
Figure 8 shows the perspective view of a representative embodiment of the guide gear and the transmission gear disposed in the inhaler device of the present invention.
Figure 9 shows the view of a representative embodiment of the blister disposed in the inhaler device of the present invention.

**Reference Numbers in Figures**
- A.: Angled Tooth
- B.: Gear
- C.: Locking arm
- a.: Steep slope tooth edge
- b.: Gradual slope tooth edge

- 1.: Inhaler device
- 2.: Locking arm/pawl
- 3.: Guide gear tooth
- 3.1: Inclined/radius surface section of the guide gear
- 3.2: Flat surface section of the guide gear
- 4.: Guide gear recess
- 5.: Blister
- 6.: Blister cavity
- 7.: Guide gear
- 8.: Outer body
- 9.: Mouthpiece
- 10.: Lid
- 11.: Inner body
- 12.: Lower housing
- 13.: Left center housing
- 14.: Right center housing
- 15.: Upper housing
- 16.: First additional gear
- 17.: Second additional gear
- 18.: Upper rolling gear of the blister lid
- 19.: Intermediate rolling gear of the blister lid
- 20.: Lower rolling gear of the blister lid
- 21.: Trigger
- 22.: Spring
- 23.: Receptacle
- 24.: Drive plate
- 25.: Transmission gear
- 26.: Clip
- 27.: Clip tab

### Detailed Description of the Invention

In the detailed description herein, a guide gear (7) that is disposed in the inhaler device (1) of the present invention is explicated in an exemplary manner referring to the attached figures only so as to clarify without introducing any limiting effects.

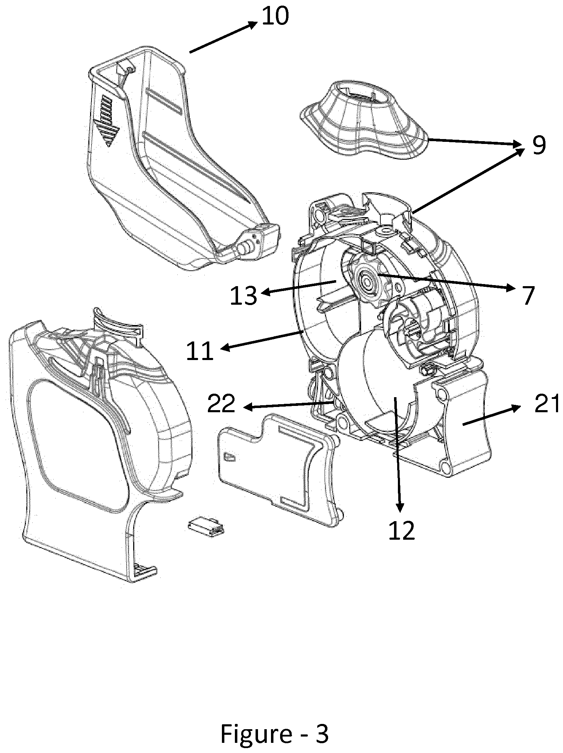

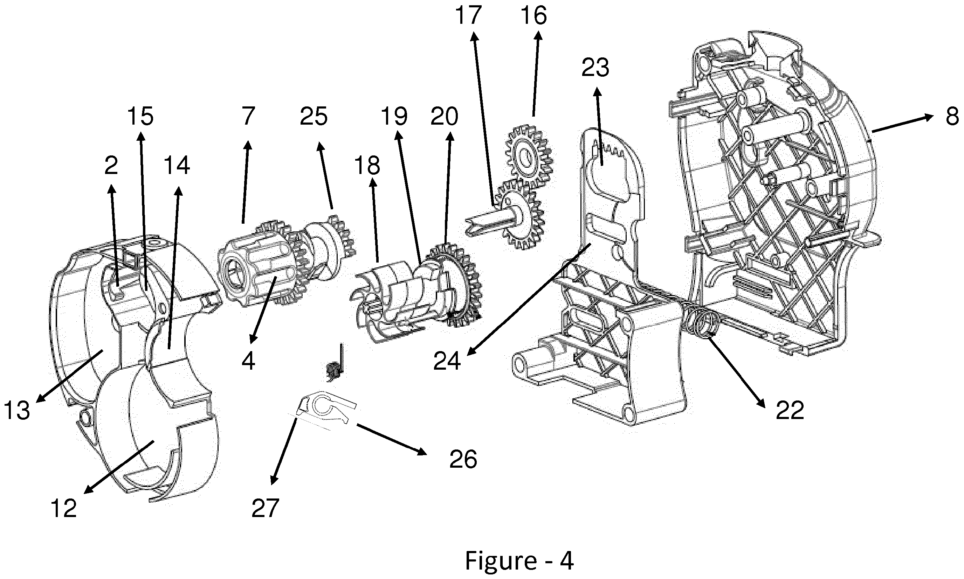

An outer body (8) of the inhaler device (1) of the present invention of which the representative view is given in Figures 3 and 4 is composed of two compatible parts with retaining tabs disposed thereon. The outer body (8) is formed by mounting said two parts to each other. A mouthpiece (9) enabling the user to inhale a medicament is provided at the upper part of said outer body (8). The mouthpiece (9) is composed of two parts, namely a section as an extension of the outer body (8) and an attachable/detachable headpiece. In order to keep the mouthpiece (9) clean and prevent foreign bodies from entering the inhaler device (1) when said inhaler device (1) is not being used, a lid (10) that pivots on the outer body (8) and that is closed on the mouthpiece (9) is provided.

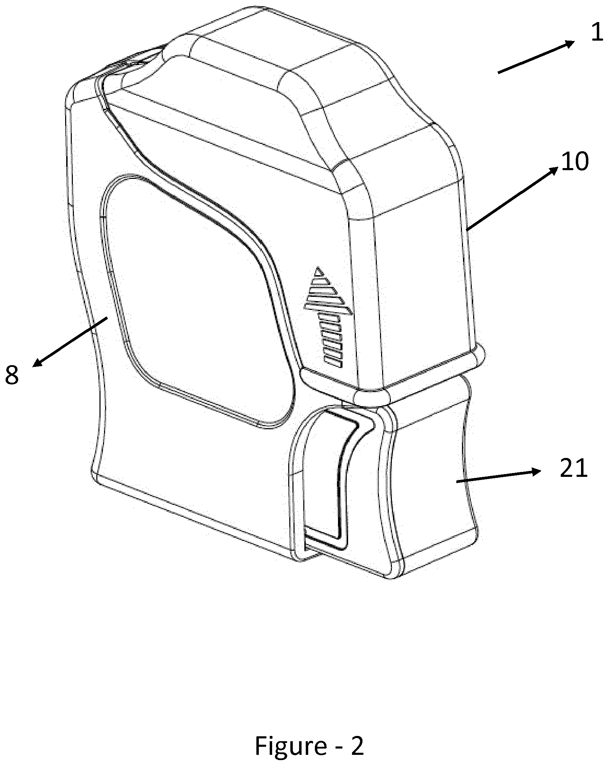

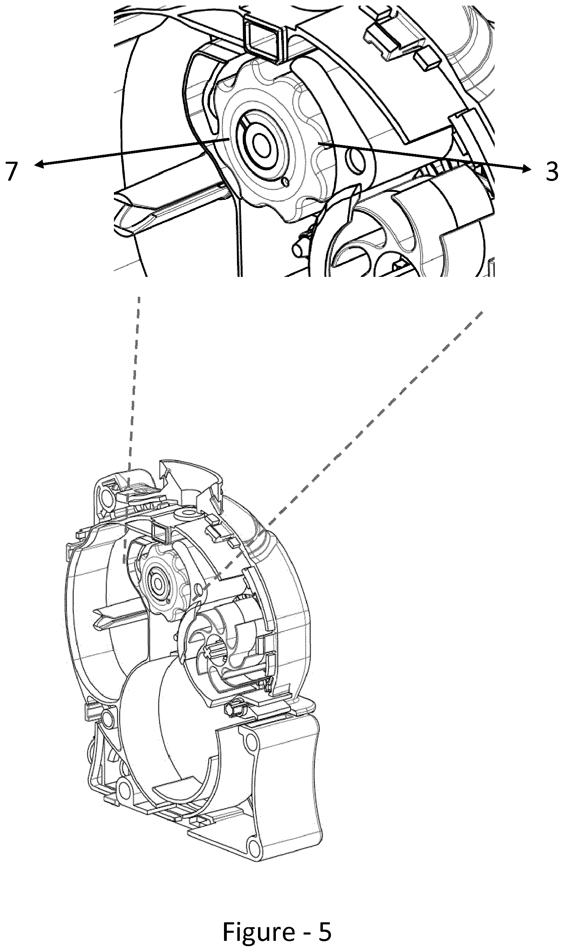

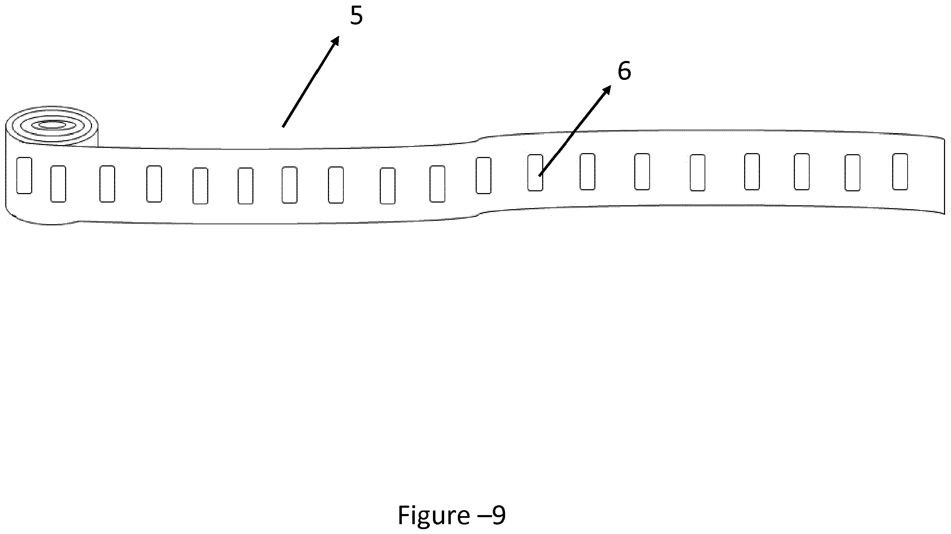

According to the representative views given in Figures 2, 3, 4, 5 and 9, a blister (5) advancement mechanism gear group is disposed on the inside of the outer body (8) so as to provide the operation of the inhaler device (1). A blister (5) having cavities (6) filled with powder medicaments is positioned along the intermediate channels of said gear group. In said gear group, just under the mouthpiece (9), a guide gear (7) is provided, wherein the blister (5) cavities (6) filled with powder medicaments are disposed into the recess (4) arranged on the body of the guide gear (7). The recesses (4) of the guide gear (7) are positioned right under the mouthpiece (9) of the device (1). An inner body (11) of which the representative view is given in Figures 4 and 5 is provided at the inner section of the outer body (8) so as to provide the positioning of the blister (5). The inner body (11) has a single-piece structure with all the lateral sides thereof being closed walls and allows the positioning of the blister (5) advancement mechanism gear group and of the blister (5). Said single-piece inner body (11) comprises a lower housing (12), a left center housing (13), a right center housing (14), an upper housing (15), recessed surfaces and tabs. The unused, full blister (5) is stored in the lower housing (12) and said strip-shaped blister (5) extends along the intermediate channels so as to be divided into two parts, namely the main layer and the lid layer, by means of the mechanism. The main layer of the blister (5), the end of which is fixed to a second additional gear (17), is stored in the left center housing (13) wherein the second additional gear (17) is disposed. The end of the lid layer of the blister (5) is fixed to a pin on an intermediate rolling gear (19).

At the beginning of the blister (5) advancement mechanism gear group of which the representative views are given in Figures 2, 3, 4 and 5, a trigger (21) is provided, that is slidably disposed at the lower part of the outer body (8). Said trigger (21) can move in the axial/linear direction at the inner section of the outer body (8). The surface on the outer section of the trigger (21) on which the user applies pressure to push said trigger (21) inwards has a concave structure so as to provide ease of use. Between said trigger (21) and the inner surface of the outer body (8) a spring (22) is provided, that is compressed (loaded) when said trigger (21) is pushed into the outer body (8). Said spring (22) has a helical form.

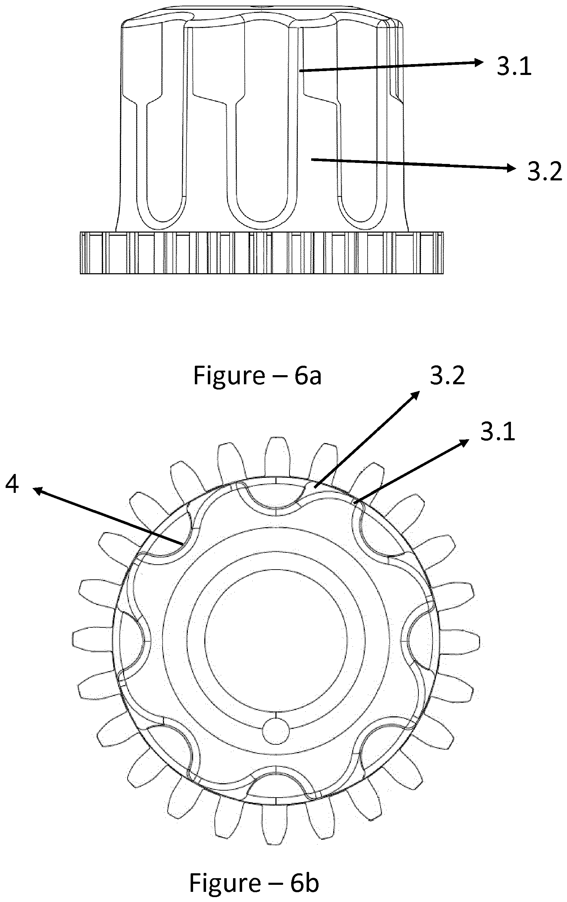

As can be seen in Figures 4, 5, 6a and 6b, in the inhaler device (1) of the present invention, a drive plate (24) is provided, that is fixed to said trigger (21) from one end and that has a receptacle (23) at the other end thereof. Said receptacle (23) is formed so as to be large enough to receive a gear therein. In the receptacle (23) of said drive plate (24), linear gears are provided, that are arranged in the axial/linear direction wherein the trigger (21) moves. The receptacle (23) is preferably rectangular and said gears are arranged at the upper edge of the receptacle (23). A transmission gear (25) is positioned in said receptacle (23). The transmission gear (25) is movably connected from the center thereof to a pin fixed to the inner body (11). Said connection allows the transmission gear (25) to make only rotational movement. A portion of the base peripheral surface of the transmission gear (25) contains teeth while the remaining portion does not have any teeth. The teeth of the transmission gear (25) engage with the linear gear of the drive plate (24). The surface of the transmission gear (25) comprising teeth in engagement with the linear gear realizes the rotation function. The surface portion without any teeth rotates inside the receptacle (23) without contacting any surfaces, thus providing an advantage in the volume and enabling the volume of the receptacle (23) to be in the acceptable range. Said transmission gear (25) engages with the guide gear (7). The guide gear (7) engages with said gear (25) so as to identically imitate the movement of the transmission gear (25).

In light of the detailed description above, the inhaler device (1) of the present invention operates in the following manner. After the opening of the lid (10), the user applies force onto the gripping surface of the trigger (21). Afterwards the trigger (21) is slid into the outer body (8). With the axial/linear movement of said trigger (21), the drive plate (24) in connection with the trigger (21) is also slid. Thus, the transmission gear (25) in engagement with the linear gear of the drive plate (24) makes rotational movement. With the movement of the transmission gear (25), the guide gear (7) disposed thereon starts rotating and rotates the gears with which the guide gear (7) engages. Thus, the blister (5) positioned on the gears is moved and said blister (5) cavities (6) are opened.

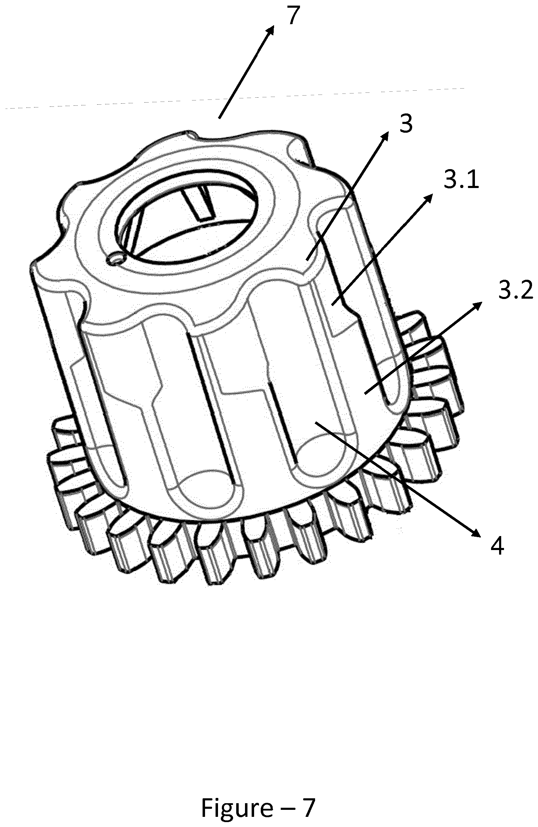

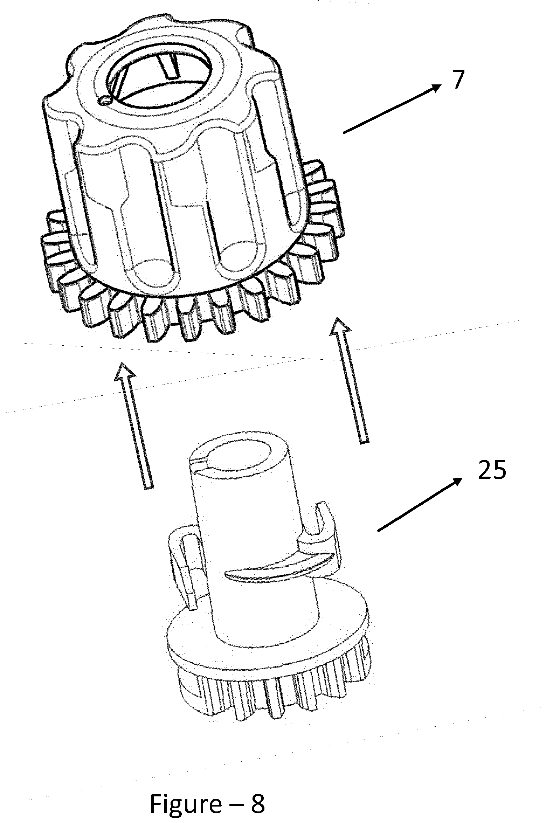

The guide gear (7) of which the representative embodiment is shown in detail in Figures 5, 6a, 6b, 7 and 8 moves unidirectionally and realizes only the forward movement of the blister (5). The transmission gear (25) in engagement with the guide gear (7) can rotate forwards or backwards.

By means of the guide gear (7), the transmission gear (25) transmits this movement to the first additional gear (16) and the second additional gear (17) as well as to the upper rolling gear (18), the intermediate rolling gear (19) and the lower rolling gear (20) of the blister (5) lid which engage with each other. While the transmission gear (25) and the guide gear (7) rotate counterclockwise, the lower rolling gear (20) of the blister (5) lid as well as the intermediate rolling gear (19) and the upper rolling gear (18) of the blister (5) lid which are positioned above the lower rolling gear (20) rotate clockwise. The blister (5) in engagement with said gears (18, 19, 20) moves through the channels of the inner body (11) and is stripped while passing through the blister (5) mechanism gear groups, thus the medicament in the blister (5) cavity (6) is exposed. The main layer of the blister (5) divided into two is rolled into the left center housing (13) and the lid layer thereof is rolled over the blister (5) lid upper rolling gear (18) that is positioned in the right center housing (14).

As a result of the trigger (21) being slid into the outer body (8), the housing arranged on the trigger (21) is fixed by engaging with a locking clip (26) tab (27) positioned right thereabove, thus providing the use of a single dose of medicament. As the user continues to make the pushing movement until said locking position, the blister (5) is enabled to be completely stripped and the proper administration of required dose of medicament is assured. As a result of said locking, the trigger (21) is fixed and the pushing member is deactivated for a short period. Moreover, said pushing movement causes the spring (22) to be compressed between the inner surface of the outer body (8) and the trigger (21).

After the user closes the lid (10) following the use of the powder medicament, the end portion of the lid (10) applies pressure onto the rear section of the locking clip (26) and lifts the end, thus separating the tab (27) of the clip (26) and the retaining housing from each other. Thus, the spring (22) in the compressed state rotates the transmission gear (25) in the reverse direction and causes the reverse movement of the mechanism. Thus, the device (1) becomes ready for reuse without the need for the user to wind up the same. However, the mechanism cannot move the blister (5) in the reverse direction. In light of the description given herein, it is clear that the inhaler device (1) of the present invention can be used only with a single pushing movement by the user. With the closing of the lid (10), the device (1) is automatically wound up and the device (1) becomes ready for reuse.

The linear movement of the trigger (21) is realized as the trigger (21) being slid in any direction including the x and y axes. Said direction is determined according to the design of the device (1).

The term "blister (5) advancement mechanism gear group" comprises the guide gear (7), the transmission gear (25), the blister (5) lid lower rolling gear (20), the blister (5) lid intermediate rolling gear (19), the blister (5) lid upper rolling gear (18), the first additional gear (16) and the second additional gear (17), and other wheels and gears that can be added to the main components of the device (1) are considered within the scope of this term.

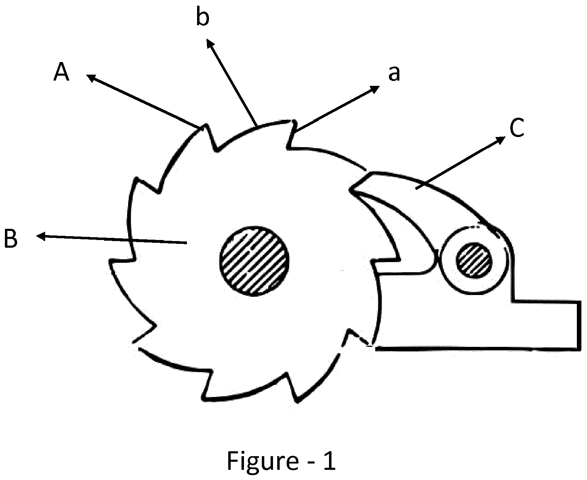

The ratchet system used in the state of the art shown in Figure 1 can be described in the following manner.

A gear (B) having angled/inclined teeth (A) comprises a locking arm (C). The inclined tooth structure constitutes the characterizing feature of said mechanism. An edge (a) of each tooth (A) forming said inclined gear (B) has a steep slope (almost right angled) structure while the other edge (b) has a gradual slope structure. At a distance in contact with the gear (B) a locking arm (C) is provided, that obstructs said teeth (A) or enables the same to move unidirectionally during the rotation of the gear (B).

In the present invention of which the representative embodiment is shown in detail in Figure 4, the dry powder inhaler device (1) is realized, comprising a circular guide gear (7) that is positioned opposite to a locking arm/pawl (2) in the advancement mechanism and wherein the blister (5) cavities (6) filled with powder medicament are disposed into the recess (4) arranged between the teeth (3) on the body of the guide gear (7). The teeth (3) of said guide gear (7) are shaped in at least two different forms. Some of the teeth (3) in said two different forms have an inclined/radius/ratchet surface (3.1) and the remaining teeth (3) have a flat surface (3.2). Thus, the guide gear (7) is enabled to operate in a controlled manner at the center and upper section thereof without wobbling out of the rotational axis. At the inner section/center of the guide gear (7) the ratchet system embodiment is realized by means of the transmission gear (25) with the tabs allowing only unidirectional rotation while the embodiment is realized by means of the inclined gear and a locking arm/pawl (2) at the outer section. Thus, the guide gear (7) is enabled to make stable rotational movement both at the center and the upper section thereof. Moreover, the upper section of the tooth section of the guide gear (7) has an inclined/radius surface (3.1) while the section close to the base has a flat surface (3.2). The gear (3) is formed as a combination of said inclined/radius surface (3.1) and the flat surface (3.2). The strip-shaped blister (5) is rolled around the flat surface (3.2) and thus during the movement of the mechanism the blister (5) is enabled to move without deviating from the movement path. An inhaler device is developed, that comprises a ratchet and a corresponding pawl engaging the ratchet to rotate in the same direction wherein an enlarged outer periphery of the teeth profile of the ratchet formed such that holding blister in a secure manner while rotation of the ratchet.

Consequently, by means of the described embodiment, an inhaler device (1) having a highly precise and smoothly operating mechanism is realized.

## Claims

1. A dry powder inhaler device (1) comprising:
- a mouthpiece (9);
- a rotatable guide gear (7) comprising a body and teeth (3), the teeth being shaped in at least two different forms (3.1, 3.2), an enlarged outer periphery of the teeth profile forming a ratchet profile, the guide gear (7) configured to receive blister cavities (6) of a strip-shaped blister (5), the blister cavities (6) filled with powder medicaments, into recess (4) arranged on the body of the guide gear (7) between the teeth (3), wherein the recess (4) of the guide gear (7) is positioned right under the mouthpiece (9); and
- a pawl (2) corresponding to the ratchet profile of the guide gear (7) and engaging the ratchet to rotate in the same direction,
the configuration of the teeth (3) of said guide gear (7) enabling in this manner holding the blister (5) in a secure manner while rotation of the ratchet and advancing the blister (5).

2. A dry powder inhaler device (1) according to Claim 1, **characterized in that** said teeth (3) having two different forms are provided with one of the flat, outward/inward oval, castellated, inclined/radius or conic forms.

3. A dry powder inhaler device (1) according to one of the preceding claims, wherein one section of said teeth (3) having two different forms has an inclined/radius surface (3.1) and the other section thereof has a flat surface (3.2).

4. A dry powder inhaler device (1) according to claim 3, wherein the longitudinal ratio of the inclined/radius surface (3.1) section of said teeth (3) to the flat surface section (3.2) is in the range of 0.1 - 9.

5. A dry powder inhaler device (1) according to claim 4, wherein the longitudinal ratio of the inclined/radius surface (3.1) section of said teeth (3) to the flat surface section (3.2) is in the range of 1 - 5.

6. A dry powder inhaler device (1) according to claim 5, wherein the longitudinal ratio of the inclined/radius surface (3.1) section of said teeth (3) to the flat surface section (3.2) is in the range of 1.3 - 3.

7. A dry powder inhaler device (1) according to claim 6, wherein the longitudinal ratio of the inclined/radius surface (3.1) section of said teeth (3) to the flat surface section (3.2) is in the range of 1.5 - 2.

8. A dry powder inhaler device (1) according to one of the preceding claims, wherein each of the teeth (3) of said guide gear (7) has the same configuration and that said teeth (3) are shaped in two or more different forms.

9. A dry powder inhaler device (1) according to one of the preceding claims, wherein the cross-sectional area of the teeth (3) of said guide gear (7) is selected from rectangular, square, oval, radius, wave forms or from the combination of these forms.

## Patentansprüche

1. Trockenpulver-Inhalationsvorrichtung (1), umfassend:
- ein Mundstück (9);
- ein drehbares Führungszahnrad (7) mit einem Körper und Zähnen (3), wobei die Zähne in wenigstens zwei verschiedenen Formen (3.1, 3.2) geformt sind,
wobei ein vergrößerter Außenumfang des Zahnprofils ein Ratschenprofil bildet,
wobei das Führungszahnrad (7) konfiguriert ist, um Blisterhohlräume (6) eines streifenförmigen Blisters (5), wobei die Blisterhohlräume (6) mit pulverförmigen Arzneimitteln gefüllt sind,
in eine Ausnehmung (4) aufzunehmen, die am Körper des Führungszahnrads (7) zwischen den Zähnen (3) angeordnet ist,
wobei die Ausnehmung (4) des Führungszahnrads (7) direkt unter dem Mundstück (9) angeordnet ist; und
- eine Sperrklinke (2), die dem Klinkenprofil des Führungszahnrads (7) entspricht und die Ratsche beaufschlagt, um sich in derselben Richtung zu drehen,
wobei die Konfiguration der Zähne (3) des Führungszahnrads (7) auf diese Weise das sichere Halten des Blisters (5) während der Drehung der Ratsche und das Vorschieben des Blisters (5) ermöglicht.

2. Trockenpulver-Inhalationsvorrichtung (1) nach Anspruch 1, **dadurch gekennzeichnet,**
**dass** die Zähne (3), welche zwei unterschiedlichen Formen aufweisen, mit einer der flachen, auswärts/einwärts, ovalen, zinnenförmigen, schrägen/radialen oder konischen Formen versehen sind.

3. Trockenpulver-Inhalationsvorrichtung (1) nach einem der vorhergehenden Ansprüche,
wobei ein Abschnitt der Zähne (3), welche zwei unterschiedlichen Formen aufweisen, eine schräge/radiale Oberfläche (3.1) und der andere Abschnitt eine ebene Oberfläche (3.2) aufweist.

4. Trockenpulver-Inhalationsvorrichtung (1) nach Anspruch 3,
wobei das Längsverhältnis des Abschnitts mit der geneigten/radialen Oberfläche (3.1) der Zähne (3) zu dem Abschnitt mit der flachen Oberfläche (3.2) im Bereich von 0,1 - 9 liegt.

5. Trockenpulver-Inhalationsvorrichtung (1) nach Anspruch 4,
wobei das Längsverhältnis des Abschnitts mit der geneigten/radialen Oberfläche (3.1) der Zähne (3) zu dem Abschnitt mit der flachen Oberfläche (3.2) im Bereich von 1 - 5 liegt.

6. Trockenpulver-Inhalationsvorrichtung (1) nach Anspruch 5,
wobei das Längsverhältnis des Abschnitts mit der geneigten/radialen Oberfläche (3.1) der Zähne (3) zu dem Abschnitt mit der flachen Oberfläche (3.2) im Bereich von 1,3 - 3 liegt.

7. Trockenpulver-Inhalationsvorrichtung (1) nach Anspruch 6,
wobei das Längsverhältnis des Abschnitts mit der geneigten/radialen Oberfläche (3.1) der Zähne (3) zu dem Abschnitt mit der flachen Oberfläche (3.2) im Bereich von 1,5 - 2 liegt.

8. Trockenpulver-Inhalationsvorrichtung (1) nach einem der vorhergehenden Ansprüche,
wobei jeder der Zähne (3) des Führungszahnrads (7) die gleiche Konfiguration aufweist und dass die Zähne (3) in zwei oder mehr verschiedenen Formen ausgebildet sind.

9. Trockenpulver-Inhalationsvorrichtung (1) nach einem der vorhergehenden Ansprüche,
wobei die Querschnittsfläche der Zähne (3) des Führungsrades (7) ausgewählt ist aus rechteckigen, quadratischen, ovalen, radialen, WellenFormen oder aus der Kombination dieser Formen.

## Revendications

1. Dispositif d'inhalateur de poudre sèche (1) comprenant :
un embout (9) ;
un engrenage de guidage (7) tournant comprenant un corps et des dents (3), les dents étant formées en au moins deux formes différentes (3.1, 3.2), une périphérie extérieure élargie du profil des dents formant un profil de cliquet, l'engrenage de guidage (7) étant configuré pour recevoir des cavités de plaquette (6) d'une plaquette (5) en forme de bande, les cavités de plaquette (6) étant remplies de médicament en poudre, dans un évidement (4) agencé sur le corps de l'engrenage de guidage (7) entre les dents (3), dans lequel l'évidement (4) de l'engrenage de guidage (7) est positionné juste sous l'embout (9) ; et
une tige (2) correspondant au profil de cliquet de l'engrenage de guidage (7) et venant en prise avec le cliquet pour tourner dans la même direction,
la configuration des dents (3) dudit engrenage de guidage (7) permettant de cette manière de maintenir la plaquette (5) d'une manière sûre pendant la rotation du cliquet et de faire avancer la plaquette (5)

2. Dispositif d'inhalateur de poudre sèche (1) selon la revendication 1, **caractérisé en ce que** lesdites dents (3) ayant deux formes différentes sont pourvues de l'une des formes plate, ovale extérieure/intérieure, crénelée, inclinée/rayon ou conique.

3. Dispositif d'inhalateur de poudre sèche (1) selon l'une des revendications précédentes, dans lequel une section desdites dents (3) ayant deux formes différentes a une surface inclinée/rayon (3.1) et l'autre section de celles-ci a une surface plate (3.2)

4. Dispositif d'inhalateur de poudre sèche (1) selon la revendication 3, dans lequel le rapport longitudinal de la section de surface inclinée/rayon (3.1) desdites dents (3) à la section de surface plate (3.2) est dans la plage de 0,1-9

5. Dispositif d'inhalateur de poudre sèche (1) selon la revendication 4, dans lequel le rapport longitudinal de la section de surface inclinée/rayon (3.1) desdites dents (3) à la section de surface plate (3.2) est dans la plage de 1-5.

6. Dispositif d'inhalateur de poudre sèche (1) selon la revendication 5, dans lequel le rapport longitudinal de la section de surface inclinée/rayon (3.1) desdites dents (3) à la section de surface plate (3.2) est dans la plage de 1.3-3.

7. Dispositif d'inhalateur de poudre sèche (1) selon la revendication 6, dans lequel le rapport longitudinal de la section de surface inclinée/rayon (3.1) desdites dents (3) à la section de surface plate (3.2) est dans la plage de 1.5-2.

8. Dispositif d'inhalateur de poudre sèche (1) selon l'une des revendications précédentes, dans lequel chacune des dents (3) dudit engrenage de guidage (7) a la même configuration et que lesdites dents (3) sont formées en deux ou plus formes différentes.

9. Dispositif d'inhalateur de poudre sèche (1) selon l'une des revendications précédentes, dans lequel la surface en section transversale transversale des dents (3) dudit engrenage de guidage (7) est sélectionnée parmi les formes rectangulaire, carrée, ovale, radiale, ondulée ou parmi une combinaison de ces formes
